# EUROPEAN PATENT APPLICATION

(11) **EP 1 870 451 A1**
(43) Date of publication of application: **26.12.2007**
(21) Application number: 05748715.9
(22) Date of filing: 12.05.2005
(51) Int. Cl.: C12N 5/06, A01N 1/02

(54) **SUPPLEMENTATION FOR EMBRYO AND/OR CELL MANIPULATION**

(30) Priority: 29.03.2005 ES 200500721
(71) Applicant: Instituto Nacional de Investigacion y Tecnologia.., E-28040 Madrid (ES)
(72) Inventor: GUTIERREZ ADÁN, Alfonso, E-28040 Madrid (ES); FUENTE MARTINEZ, Julio de la, E-28040 Madrid (ES); MOREIRA, Pedro, E-28040 Madrid (ES); PALASZ, Andre, E-28040 Madrid (ES)
(74) Representative: Gonzalez Palmero, Fé
(86) International application number: PCT/ES2005/000255
(87) International publication number: WO 2006/103300

(57) **Abstract**

The question is to increase the quality and safety of the media used in embryo and cell manipulations by means of supplementation, in the manipulation medium, by one or several of the following compounds: synthetic hyaluronan (sHA), phospholipids or unsaturated fatty acids obtained from soybean seeds (PLFA), replacing others which are habitually added to embryo manipulation media and which produce potential damage and/or contamination by viruses, prions, endotoxins, etc., this being important with regard to the quality of the embryos or cells which it is desired to generate, both for the production of transgenic animals, for animal production, and for cell therapy or assisted reproduction; achieving a reduction in adhesiveness and an increase in viscosity, without losing the fluidity of the medium; this is essential in micro-manipulations such as ICSI, nuclear transfer, embryo biopsies, the micro-injection of cells into embryos in pre-implantational stages, or cell fusion.

## Description

### Object of the Invention

This invention relates to a system for increasing the quality and safety of the media used in embryo and cell manipulation, said manipulation medium being formed with one or several of the following compounds: synthetic hyaluronan (sHA), phospholipids or unsaturated fatty acids obtained from soybean seeds (PLFA), generating by this means high-quality embryos and cells, both for the production of transgenic animals, for animal production, and for cell therapy or for assisted reproduction.

The object of this invention is, therefore, to provide supplements in order to produce an optimal medium for facilitating embryo and cell micro-manipulations, of great usefulness in Biology, Biomedicine and Biotechnology, improving the embryo/cell manipulation media, which produces embryos and/or cells of higher quality and improves the percentage of embryo implantation and the subsequent survival of said embryos or cells.

### Background of the invention

For many techniques of embryo/cell manipulation, media are required which feature a high viscosity and at the same time sufficient fluidity to allow control over the movement of the fluid which accompanies the cells being manipulated, and which prevent the cells manipulated from adhering to the culture plate, to each other or to other molecules in the medium.

The macromolecules normally used in manipulation media can either be obtained from blood, such as serum or seric albumin, which entail a potential source of contamination (viruses, prions, endotoxins), or can be synthetic macromolecules, such as polyvinylpyrrolidone (PVP), polyvinylalcohol (PVA), Ficoll, etc., which do not diffuse from the cell interior and which are not digested by the cell lysosomal enzymes, as a result of which they are retained in the interior of the cells after the embryo/cell manipulations.

Although apparently these synthetic molecules are harmless, they are elements which are not present in any animal cells, and in several cases it has been documented that they can be toxic for cell viability. For example, it has been determined that PVP can interfere with the nucleus decondensation which the sperm must perform in order to carry out a correct fertilisation (Dozortsev et al., 1995. Sperm plasma membrane damage prior to intracytoplasmic sperm injection: a necessary condition for sperm nucleus decondensation. Human reproduction 10, 2960-64). Highly negative effects have been observed in mouse embryos when the sperms were incubated with PVP prior to fertilisation (Mizuno et al., 2002. Fertilization and embryo development in mouse ICSI model using human and mouse sperm after immobilization in polyvinylpyrrolidones. Human reproduction 17, 2350-55).

The use of Percoll with PVP-coated silica particles has recently been banned in human assisted reproduction, due to the risk of contamination by endotoxins and to the alterations produced in the membrane of the gametes; for this reason the gametes must be washed thoroughly in order to eliminate the Percoll-PVP, which contributes to a detriment in the quality of the gametes. However, PVP continues to be used in some micro-manipulations such as in ICSI, although it is essential to produce new alternatives.

### Description of the invention

The supplements for embryo and cell manipulation media proposed by the invention resolve the aforementioned problems in the various aspects mentioned, in an entirely satisfactory manner, as they comprise an optimal medium for facilitating embryo/cell micro-manipulations, and do not cause the potential damage and/or contamination regarding viruses, prions, endotoxins, etc.

More specifically, this invention provides supplements for producing an optimal medium for facilitating embryo and cell micro-manipulations in human and animal assisted reproduction, for example in intracytoplasmic injection (ICSI), in nuclear transfer, with both therapeutic and reproductive ends, in the case of non-human animals, in the production of non-human transgenic animals, such as the injection of stem cells into preimplantational embryos, to produce chimeras, in the production of transgenic animals by means of ICSI, in cell manipulations used in transgenesis or in cell therapy, in embryo biopsies, etc.

The supplementation proposed by the invention consists of the use of one or several of the following compounds: synthetic hyaluronan (sHA), phospholipids or unsaturated fatty acids obtained from soybean seeds (PLFA), as a medium for embryo and cell micro-manipulation.

Hyaluronan is a natural polymer formed by repetitions of N-acetylglucosamine disaccharide and D-glucoronic acid units, and is distributed throughout the organism. It is generally obtained by the continuous bacterial fermentation of *Streptococcus equi* (Cifonelli JA, Dorfman A. Biosynthesis of Hyaluronic acid by group A Streptococcus: the uridine nucleotides of groups a. Streptococcus. J. Biological Chemistry 1957; 288:547-557).

The supplementation of the medium of this invention combines the presence of sHA or PLFA in any proportion. The medium supplement may be added to any embryo or cell medium, for example to media buffered with bicarbonate, HEPES or MOPS. This invention contemplates any combination of the proportions of phospholipids and fatty acids present in PLFA.

When the presence of hyaluronan and of unsaturated fatty acids obtained from soybean seeds is combined, the conditions of viscosity and fluidity of the medium can be modified, likewise the characteristics of adhesiveness, in such a way that, depending on the needs of the micromanipulator, the viscosity and fluidity can be adjusted without losing the characteristics which prevent the embryos and/or cells from adhering to the culture plate and being impossible to manipulate correctly.

By means of supplementing the manipulation medium with sHA and/or PLFA, a reduction in adhesiveness and an increase in viscosity, without losing the fluidity of the medium, is achieved; this is essential in micro-manipulations such as ICSI, nuclear transfer, embryo biopsies, the micro-injection of cells into embryos in pre-implantational stages, or cell fusion. This increase in viscosity which is produced allows the exact control of the flow of the medium through the manipulation pipettes, avoiding the inclusion into said medium of any other product which might affect the viability of the mammal cells. Besides, hyaluronan features ion-chelating characteristics which protect the cells from cell or nuclear damage; it is a physiological molecule, present in the female genital tract, which is easily eliminated by the cells by means of lysosomal enzymes, it diffuses easily from the cell interior, prevents the embryos or cells from adhering to the culture plates or to the micro-tools used in embryo manipulation, and not only does it not have any negative effect on the development of the embryo, but it also improves the quality of the embryos which result from the manipulation, and the levels of implantation. PLFA and/or sHA represent an effective, physiological alternative to the macromolecules used in manipulation media in order to modulate the micro-manipulations performed on mammal embryos/cells.

The chelating activity of sHA protects the preparations of sperms and embryos/cells which, due to damage to the membrane, either caused by the freezing processes, or by endogenic processes, free endonucleases which degrade the DNA of the sperm. It has been observed that thanks to the presence of sHA, an inhibition of these endonucleases is produced due to the capture of ions which are essential for the working of these endonucleases. Besides, the motility of the sperms increases, probably due to the capture of zinc ions, producing an effect similar to that described in human sperms, where it has been proved that the presence of chelating agents increases the motility of the sperms.

Hyaluronan and phospholipids or unsaturated fatty acids obtained from soybean seeds display an antioxidant activity in such a way that they protect embryos and cells from the damage caused by the oxygen free radicals. In order to produce high-quality embryos/cells, the oxidative damage caused by these radicals must be reduced. The first consequence of the aforementioned radicals is the fragmentation of the DNA, which causes embryo mortality and, in some cases, infantile tumours.

When the sperms were manipulated in the presence of sHA instead of PVP, a greater number of blastocysts were produced, of a higher embryo quality, and furthermore, these blastocysts were implanted with a higher frequency.

Hyaluronan is used to improve the *in vitro* culture conditions, such as in the case of pig embryos cultured in NCSU-23 medium. Similar results have been obtained with mouse oocytes and with bovine embryos.

It has also been proved that supplementation of the medium with phospholipids accelerates the acrosomal response of human sperm. When added to the *in vitro* culture of, for example, bovine embryos in the place of foetal serum, high-quality embryos are produced, which undergo perfectly the freezing and thawing processes, eliminating the risks which the use of foetal serum entails.

The elimination of blood-derived supplements such as serum is essential for the international transport of embryos. It also allows culture conditions to be standardised as, when using biological proteins containing macro-molecules in variable quantities, and whose concentration varies from lot to lot, standard conditions can never be guaranteed.

### Embodiments of the invention

The examples described below represent the particular embodiments of the invention, and are not intended to limit the scope of its protection in any way.

### Example 1: Production of ICSI in mice, replacing the PVP in the manipulation medium with hyaluronan. Obtaining and freezing the sperms.

The sperm in the epididymis is collected and uniformly resuspended in M2 medium. The sperm cells collected are washed by centrifuging in a 1.5 ml propylene centrifuge tube with 1 ml of fresh medium, and resuspended to obtain a final concentration of 1-2 million sperms/ml. Subsequently, aliquots of 60-70 microlitres of sperm suspension in cryogenic tubes (NUNC, Copenhagen) are made; these tubes are correctly closed and placed directly in liquid nitrogen at -196°C during 10-15 minutes, without immersing them completely, in order to prevent contamination of the samples with liquid nitrogen. The samples are subsequently stored during periods of up to 4 weeks at -80°C, avoiding their thawing during the transition from -196°C to -80°C. Sterility was maintained during the entire procedure.

The sperm samples are thawed at ambient temperature during 10 minutes prior to their use. They are subsequently diluted in 10% PVP or in 80% hyaluronate (hyaluronic acid) in M2, solutions with similar viscosities. This final sperm solution must be micro-injected at ambient temperature during the next two hours.
Intracytoplasmic micro-injection of the sperm nucleus.

During the fertilisation of oocytes in metaphase II by intracytoplasmic sperm injection (ICSI), the complete sperm may be injected. However, injection of the tail of the male gamete must be avoided if its centrosome does not take part in the fertilisation process (i.e. in mice). This facilitates the micro-injection process and reduces the volume of fluid injected into the oocyte cytoplasm. Micro-injection may be carried out in the conventional way, [i.e. the method for hamster is described in Yanagida, K., Yanagimachi, R., Perrault, S.D. and R.G. Kleinfield, Biology of Reproduction 44, 440-447 (1991); Perry AC, Wakayama T, Kishikawa H, Kasai T, Okabe M, Toyoda Y, Yanagimachi R. Mammalian transgenesis by intracytoplasmic sperm injection. Science. 1999 May 14;284(5417):1180-3.], or with the aid of a piezoelectric micro-injection unit (Piezo Impact Drive Unit by Prime Tech Limited, Tsukuda, Ibaraki-Ken, Japan). This unit uses the piezoelectric effect to move the injection pipette to and fro small distances, approximately 0.5 micrometres, in a controlled manner. The unit allows the duration and the intensity of each pulse to be regulated.

In order to inject into the oocyte a sperm nucleus mixed with PVP or hyaluronic acid, the tail of the sperm, if it has one, is first drawn into the injection capillary. This capillary must have a point, straight in cross-section if a piezoelectric unit is used, with an internal diameter of approximately 6-8 µm, depending on the species in question. In some species, for example in the mouse, injection of the sperm head is sufficient for normal embryo development, and this simplifies the micro-manipulation process, in such a way that it is possible to inject only the heads, separated during the freezing-thawing process, which are also those which have probably suffered the most in the freezing-fragmentation process.

During intracytoplasmic injection, the oocyte is placed by means of negative pressure and with the help of a holding pipette, with its metaphase plate distant from the point of penetration, after 6 or 12 hours. The pellucid zone is perforated by the application of piezoelectric pulses with sufficient intensity and speed. After ejecting the piece of the zone which remains in the point of the injection pipette, in the micro-manipulation droplet or in the periviteline space of the oocyte, the head of the sperm is brought near to the opening of the injection pipette. The injection pipette is moved forwards, as far as two-thirds of the diameter of the oocyte and there, a single minimum-intensity pulse is applied so that its membrane will open. The relaxation of the oocyte membrane indicates its penetration. The sperm is released with a minimum quantity of fluid, no more than approximately 6 picolitres, into the cytoplasm of the oocyte, thus carrying the transgene of interest into its interior. The injection pipette is subsequently withdrawn gently from the cytoplasm of the oocyte in order to prevent the risk of cell lysis. Micro-injection should be carried out as rapidly as possible, in groups of 10-15 oocytes which, after 10-15 minutes' recovery in the micro-injection droplet, are placed in culture conditions (i.e. KSOM medium + amino acids for mouse oocytes, equilibrated at 5% CO₂ and 37°C).
Development of the manipulated embryos for the production of offspring.

The embryos developed *in vitro* to the stage of 2-8 cells, unhatched blastocyst or morula, are transferred to the oviduct or the uterus of a pseudopregnant female. In mice, between 15 and 20 embryos are transferred per recipient animal.

In an experiment carried out in mice, a total of 239 oocytes were injected with sperms resuspended in M2 medium supplemented with 10% PVP (n 115) or 80% HA (n124). When ICSI was carried out with HA, 29 of 35 blastocysts transferred (83%) implanted themselves. This proportion of implantation was considerably higher (p<0.05, Chi-square) than that observed in the group of ICSI with PVP, in which only 19 of the 35 blastocysts transferred (54%) managed to implant themselves. Bearing these results in mind, it may be concluded that the use of HA for ICSI in mice could be a better alternative than PVP. It could be even more beneficial if the adhesiveness of the sperms within the micropipette is eliminated and if implantation rates higher than those achieved with PVP are maintained.

### Example 2: The presence of sHA in the micro-manipulation medium for performing ICSI in mice prevents the nuclear fragmentation of the sperms.

As may be observed in the methodology of ICSI in mice, quoted in Example 1, the implantation results are very low, probably due to the fragmentation produced in the DNA of the sperm during the manipulation necessary for the performance of ICSI. It has been described that the increase in oxygen free radicals and the presence of sperm endonucleases, which are freed as a consequence of the cell damage, bring about the fragmentation of the sperm DNA. This sperm with damaged DNA cannot be recognised easily, and it is therefore micro-injected into the oocyte, giving rise to a non-viable embryo or a foetal loss, depending on the degree of nuclear fragmentation. The sperms of five mice which were to be injected (ICSI) into oocytes with M2 in the presence of PVP or sHA were processed. The sperms are frozen and thawed as described in Example 1. They are subsequently maintained at 22°C during 2 hours and the degree of fragmentation of the sperm nucleus is determined; it may be seen that in the absence of sHA there was an average of 45% fragmentation, while in the presence of sHA this percentage was reduced to 25%.

### Example 3: Replacement of the foetal serum of animal origin by PLFA in the culture medium and freezing of bovine embryos:

Bovine embryos are produced from superovulated cows. Seven days after insemination these embryos are collected by means of the uterine washing of 20 cows, using two media, either PBS supplemented with 5% FCS, or PBS supplemented with 0.3% PLFA. The embryo collection percentage, calculated in accordance with the total of embryos collected by corpora lutea observed, is similar for the two washing media used (Table 1).

The embryos previously collected are placed in TCM-199 medium, buffered with HEPES and supplemented with 5% FCS or with 0.3% PLFA. After 2 hours' exposure at 22°C, the embryos are transferred to culture droplets and are placed in incubators at 39°C with 5% CO₂, 5% O₂ and 90% N₂. After 24 and 48 hours the proportion of embryos which develop to become blastocytes and which hatch correctly is determined (Table 2). No differences have been observed between the development of the groups of embryos cultured with TCM-199 supplemented with 5% FCS or with 0.3% PLFA.

In a third experiment, embryos developed over 7 days are frozen in order to study their cryotolerance. First, the embryos are maintained at a temperature of 22°C during one hour in the medium containing FCS or PLFA. Subsequently the embryos are equilibrated for 5 minutes at 22°C in 1.5M ethylene glycol and 0.3% sucrose in PBS supplemented with 0.5% BSA. Finally the embryos are inserted in 0.25 ml straws and frozen using standard equilibration procedures. Thawing is carried out in a bath at 30°C, and subsequently the post-freezing survival percentage and subsequent development *in vitro* is quantified (Table 3).

**Table 1. Recovery of embryos with PBS containing FCS or PLFA.**

| Treatment | No. of animals | Total no. of Corpora Lutea | Total no. of embryos | Collection percentage (%) |
|---|---|---|---|---|
| PBS + FCS | 10 | 190 | 104 | 54.7 |
| PBS + PLFA | 10 | 219 | 117 | 53.4 |

**Table 2. Development of 7-day bovine embryos in TCM-119 medium supplemented with FCS or PLFA.**

| Treatment | No. of blastocytes | No. of live embryos 48h (%) | Embryos hatched 72h (%) |
|---|---|---|---|
| TCM-199 + FCS | 37 | 33/37 (89.2) | 15/33 (45.4) |
| TCM-199 + PLFA | 41 | 38/41 (92.7) | 16/38 (42.1) |

**Table 3. Survival and development of bovine embryos cultured and frozen in medium containing FCS or PLFA.**

| Treatment | No. of embryos recovered/frozen | No. of live embryos 48h (%) | Embryos hatched (%) |
|---|---|---|---|
| TCM-199 + FCS | 25/25 | 18/25 (72.0) | 7/18 (38.8) |
| TCM-199 + PLFA | 26/27 | 21/26 (80.7) | 9/21 (42.8) |

## Claims

1. Supplementation for embryo and/or cell manipulation media, both for the production of transgenic animals, for animal production, and for cell therapy or assisted reproduction, **characterised in that** it is comprised of one or several of the following compounds: synthetic hyaluronan (sHA), phospholipids or unsaturated fatty acids obtained from soybean seeds (PLFA), which prevent the potential damage and/or contamination with regard to, for example, viruses, prions or endotoxins.

2. Supplementation for embryo and/or cell manipulation media, according to claim 1, **characterised in that** it combines the presence of sHA and PLFA in any proportion.

3. Supplementation for embryo and/or cell manipulation media, according to preceding claims, **characterised in that** it contemplates any combination of the proportions of phospholipids and fatty acids present in the PLFA.

4. Supplementation for embryo and/or cell manipulation media, according to preceding claims, **characterised in that** the medium supplement may be added to any cell or embryo medium, for example to media buffered with bicarbonate, HEPES or MOPS.

5. The use of the synthetic hyaluronan (sHA), phospholipids or unsaturated fatty acids obtained from soybean seeds (PLFA) of the preceding claims, in order to eliminate adhesiveness and to increase the viscosity and fluidity for the production and preparation of media which allow the *in vitro* manipulation of embryos prior to their implantation in the uterus.

6. The use of the synthetic hyaluronan (sHA), phospholipids or unsaturated fatty acids (PLFA) of the preceding claims, in order to eliminate adhesiveness and to increase the viscosity and fluidity for the production and preparation of media which allow the *in vitro* manipulation of somatic and embryo cells.

7. The use of synthetic hyaluronan (sHA), phospholipids or unsaturated fatty acids (PLFA), according to preceding claims, in order to protect the embryos/cells from the action of oxygen free radicals and to improve their survival and their subsequent implantation in the uterus.

8. The use of synthetic hyaluronan (sHA), phospholipids or unsaturated fatty acids (PLFA), according to preceding claims, in order to inactivate cell endonucleases which degrade nuclear DNA.

9. The use of synthetic hyaluronan (sHA), phospholipids or unsaturated fatty acids (PLFA), according to preceding claims, in order to standardise culture conditions with macromolecule supplements with biological functions.
